(19) Europäisches Patentamt

European Patent Office

Office européen des brevets

(11) **EP 0 879 050 B1**

(12) **EUROPEAN PATENT SPECIFICATION**

(45) Date of publication and mention
of the grant of the patent:
**20.11.2002 Bulletin 2002/47**

(21) Application number: **97943083.2**

(22) Date of filing: **10.09.1997**

(51) Int Cl.⁷: **A61K 9/20**

(86) International application number:
**PCT/HU97/00051**

(87) International publication number:
**WO 98/010753 (19.03.1998 Gazette 1998/11)**

(54) **CAPTOPRIL TABLETS**

CAPTOPRILTABLETTEN

COMPRIMES DE CAPTOPRIL

(84) Designated Contracting States:
**AT BE CH DE DK ES FI FR GB GR IE IT LI LU MC NL PT SE**
Designated Extension States:
**LT LV RO SI**

(30) Priority: **10.09.1996 HU 9602470**

(43) Date of publication of application:
**25.11.1998 Bulletin 1998/48**

(73) Proprietor: **EGIS GYOGYSZERGYAR RT.**
**1106 Budapest (HU)**

(72) Inventors:
 • **FEKETE, Pál**
   **H-1051 Budapest (HU)**
 • **PALFI, Zoltánné**
   **H-1172 Budapest (HU)**
 • **KRISZTIAN, Mária**
   **H-1161 Budapest (HU)**
 • **GORA, Lászloné**
   **H-2117 Isaszeg (HU)**
 • **LADY, Blanka**
   **H-1138 Budapest (HU)**
 • **SZENTGROTI, Pálné**
   **H-1071 Budapest (HU)**

(74) Representative: **Beszédes, Stephan G., Dr.**
**Patentanwalt**
**Postfach 11 68**
**85201 Dachau (DE)**

(56) References cited:
**EP-A- 0 418 582          EP-A- 0 426 066**
**US-A- 5 030 648          US-A- 5 158 777**

 • **J.P.REMON ET AL.: "Effect of raw materials and processing on the quality of granules prepared from microcristalline cellulose-lactose mixtures" DRUG DEV. IND. PHARM., vol. 13, no. 1, 1987, pages 1-14, XP002052285**

## EP 0 879 050 B1

**Description**

Background of the invention

This invention relates to tablets containing captopril as active ingredient and a process for the preparation thereof.

It is known that (S)-1-(3-mercapto-2-methyl-1-oxo-propyl)-L-proline (International Non-propriatory Name, INN, "captopril") is a competitive ACE inhibitor, decreases arterial and venal vascular tone, the complete periferial vascular resistance, the cardial pre- and post-loading and increases the minute-volume. Preparation of captopril is disclosed in Hungarian patent No. 181,965. The active ingredient content of captopril tablets used in therapeutical practice amounts to 6.25-100 mg and the daily dosage is 6.25-300 mg. Captopril is highly sensitive to oxidation and captopril disulfide is formed on the thiol group in a free radical oxidation process. In solid pharmaceutical compositions the velocity of decomposition is increased during the drying step of the manufacturing process and also by the moisture content of the composition. In the preparation of captopril compositions particular care is to be taken to select a suitable auxiliary agent system and manufacturing process, respectively, which induces decomposition and oxidation of the active ingredient to the smallest extent.

It is well-known to the skilled art worker dealing with formulation that captopril is susceptible to decomposition. Several patent specifications are directed to this problem. According to Japanese patent № 86 36,127 the stability of active ingredients comprising a thio group - including captopril - may be improved by using ascorbic acid, sodium ascorbate, erithorbinic acid or the sodium salt thereof, sodium bisulfite, sodium sulfite and/or sodium metabisulfite. According to Japanese patent № 82 112,367 in injectable solutions the stability of captopril may be augmented with the aid of amino acids and salts thereof. Japanese patent № 94 32,776 relates to the use of antioxidants as well. According to US patent № 5,158,777 the simultaneous use of disodium edetate and ascorbic acid is suggested. However, the use of the above antioxidants - with the exception of ascorbic acid - is accompanied by sanitary risks because said substances may often cause allergic symptoms. However according to the disclosure of US patent № 5,158,777 ascorbic acid failed to exhibit satisfactory stabilising effect when used per se.

Another problem emerging in the therapeutical use of captopril compositions is that the individual dose of the compositions varies to a large extent and that in chronical treatment the applicable dose can only be adjusted by gradual increase of the dosage. In order to provide flexible dosage of pharmaceutical compositions containing captopril which can be readily adjusted in view of the individual requirements of the patients, there is a strong need for tablets which have a variable active ingredient content within wide limits one the one hand and can be broken to two parts (halved) on the other. Constant active ingredient delivery requires that compositions having different active ingredient content should be made available with identical percentual composition on the one hand, and that when splitting the composition the active ingredient content should be substantially the same in each part of the tablet on the other. It is very important that the manufacturing process should ensure high homogenity of the active ingredient and the auxiliary agents in the composition.

The known processes for the manufacture of pharmaceutical compositions containing captopril fail to comply completely with the above requirements. In European patent publication EPA № 288,732 captopril containing pellets filled into capsules and prepared by extrusion and spheronization are disclosed. Such pellets contain 3-60 % of the active ingredient and 5-50 % of a carboxylic acid facilitating pellet formation.

The preparation of oral osmotic compositions is disclosed in PCT WO 91/01,130. This composition is a two-layered tablet coated, with a semipermeable layer. One layer contains the active ingredient and the other layer comprises a water-swellable polymer, while a bore is drilled into the layer containing the active ingredient through which the active ingredient is delivered into the organism.

The preparation of zero order sustained release coated tablets is disclosed in US patent specification № 4 756 911. The tablet core contains at least 70 % of active ingredient and 5-15 % of a hydrocolloidal gel forming auxiliary agent, particularly hydroxypropyl methyl cellulose, and the coating of the tablet consists of a 4:1 - 1:4 mixture of at least one hydrophlic polymer and at least one hydrophobic polymer.

The preparation of compositions comprising gel forming auxiliary agents (e.g. a polymer comprising a lactame group and a polymer comprising a carboxy group) and a gas forming auxiliary agent (e.g. sodium hydrogen carbonate) is disclosed in European patent publication № EPA 669,129.

Sustained release capsules comprising a semisolid filling, which consists of at least one fatty acid glyceride and/or polyethylene glycol ester, are disclosed in US patent № 5,433,951.

Summary of the invention

It is the object of the present invention to provide captopril tablets which are highly stable and show a high homogenity of the active ingredient and the auxiliary agents.

The above object is achieved by selecting a special filler/binding agent combination and a specific ratio of filler and binding agent.

According to the present invention there are provided captopril tablets comprising a filler and binding agent, a disintegrating agent, a lubricant and a glidant agent wherein the filler and binding agent is a 1.5:1 - 1:1 weight ratio mixture of lactose and microcrystalline cellulose and the total amount of said mixture is 60-80 % by weight of the total weight of the tablet.

According to the present invention there is also provided a process for the preparation of captopril tablets containing a filler and binding agent, a disintegrating agent, a lubricant and a glidant agent which comprises using as filler and binding agent a 1.5:1 - 1:1 mixture of lactose and microcrystalline cellulose, the amount of said mixture being 60-80% by weight related to the total weight of the tablets.

Detailed description of the invention

The present invention is based on the recognition that captopril tablets having high stability can be prepared by adjusting the ratio of lactose and microcrystalline cellulose in the tablets to a value between 1.5:1 and 1:1.

The above recognition is so much the more surprising as on the basis of the state of the art it could have been expected that high stability of captopril can be achieved by using a system of auxiliary agents which has the lowest moisture content and the smallest moisture-uptake.

It can be seen from the comparative experiments disclosed in the present patent specification that on increasing the amount of the microcrystalline cellulose having a high moisture-uptake, also the moisture-uptake of the tablets increases. It has been found, however, in an unforeseen manner, that when using a relatively narrow interval of lactose/microcrystalline cellulose ratio, the amount of the captopril disulfide formed in the captopril tablets shows a minimum and tablets of high stability are obtained.

The total lactose and microcrystalline cellulose content of the captopril tablets of the present invention is 60-80 % by weight, preferably 65-75 % by weight, particularly 70 % by weight, related to the total weight of the composition.

The active ingredient content of the tablets according to the present invention is preferably 5-150 mg. The particle size of 90 % of the active ingredient is preferably 3-80 $\mu$m and the starting captopril disulfide content is preferably below 0.3 % by weight.

Lactose is used preferably in the form of lactose monohydrate. The amounts and % values used in the present patent specification are expressed in lactose monohydrate.

It is preferred to use lactose monohydrate prepared by spray-drying and having an average particle size of 50-200 $\mu$m, particularly 80-160 $\mu$m.

The particle size of 90 % the microcrystalline cellulose used in the compositions of the present invention is 20-150 $\mu$m, particularly 50-100 $\mu$m.

The compositions of the present invention may contain as disintegrating agent preferably corn starch, carboxymethyl starch, partially hydrolized starch, carbomethyl cellulose or cross-linked polyvinyl pyrrolidone. The amount of said disintegrating agent is preferably 1-20 % by weight, particularly 5-15 % by weight, related to the total weight of the composition.

The compositions of the present invention contain as lubricant preferably stearine, magnesium stearate, calcium stearate, sodium stearine fumarate or hydrogenated castor oil. The amount of the glidant agent is 0.2-3 % by weight, preferably 0.5-2 % by weight, related to the total weight of the composition.

The compositions of the present invention contain preferably silica as glidant agent . The amount of this auxiliary agent is 0.2-1 % by weight, preferably 0.4-0.6 % by weight.

The auxiliary agents - disintegrating agents, glidant agents and lubricants meet the criteria disclosed in the Pharmacopoeia (e.g. USP 23 or VII. Hungarian Pharmacopoeia).

The tablets according to the present invention may be prepared preferably by the so-called direct compression technology which has the advantage that the drying step is eliminated. According to this process prior to the tabletting step the powder of the active ingredient is homogenized in solid state with auxiliary agents which ensure suitable compressibility of the composition.

The stability of the captopril tablets of the present invention is very good. If broken one into two or four parts, the standard deviation of the active ingredient content of the parts thus obtained is better than the corresponding values of known compositions.

Further details of the present invention are to be found in the following Examples without limiting the scope of protection to the Examples.

In comparative Example 1 the effect of the ratio of lactose monohydrate and microcrystalline cellulose on the compressibility of the powder blends is examined. The measured data are in conformity with the teaching of the state of the art and show that on increasing the amount of microcrystalline cellulose used as dry binder, the powder mixture can be more easily pressed i.e. on using identical pressing force the breaking strength of the tablets becomes higher and the friability gets lower.

In comparative Example 2 the effect of the ratio of lactose and microcrystalline cellulose on the moisture-uptake of the tablets is examined. The results are in conformity with the teaching of prior art and show that when stored under a relative humidity of 75 % the increase of the amount of the microcrystalline cellulose having a higher moisture-uptake (higher equilibrum moisture content) results in an increase of the moisture-uptake of the tablets.

In comparative Examples 3 and 4 the effect of the ratio of lactose and microcrystalline cellulose on the stability of the active ingredient content of the tablets is examined. In view of the state of the art the results are surprising and show in an unforeseen manner that when storing the tablets under 75 % relative humidity, oxidation of captopril - i.e. the captopril disulfide content of the tablets - shows a minimum at a lactose monohydrate : microcrystalline cellulose ratio interval of 1.5-1 : 1-1. On the basis of prior art it could have been expected that tablets containing the lowest amount of microcrystalline cellulose would be the most stable. The series of experiments show, however, that if the ratio of lactose monohydrate and microcrystalline cellulose is adjusted to a relatively narrow interval, the amount of captopril disulfide formed in the tablets is reduced to a minimum and this value is below the captopril disulfide content of captopril tablets allowed by Pharmacopoeia (e.g. USP 23).

In Examples 5-8 the preparation of captopril tablets of varying composition according to the present invention is described.

In comparative Example 9 the standard deviation of the individual active ingredient content of tablets manufactured on industrial scale and the standard deviation of the individual active ingredient content of parts of tablets split in two or four parts, respectively is disclosed. The experimental results show that if the captopril tablets of the invention are split in two or four parts, respectively, the standard deviation of the active ingredient content of said tablet parts is very favourable. Accordingly the pharmaceutical composition of the present invention can be preferably used because such compositions meet the special requirements of captopril therapy i.e. when on adjusting the individual dosage required by a patient the dose is to be increased, the tablets according to the present invention can be reliably split in two or four parts due to the low standard deviation of the active ingredient content of the tablet parts.

In comparative Example 10 the disulfide content of samples subjected to loaded stability test is determined. Tablets containing 12.5 mg of captopril are packed into blisters formed from a PVC/PVDC plastic foil and closed by an aluminium foil. The tablets of the present invention are compared to tablets which have a lactose/microcrystalline cellulose ratio outside the scope of the present invention but are otherwise of identical composition. The blistered tablets are stored at 40°C under a relative humidity of 75 % for 3 months. After this storing period the captopril disulfide content of the invention tablets increases from the initial value of 0.31 % to 3.32 %, while in the reference tablets the captopril disulfide content augments from 0.30 % to 5.04 %.

Example 11 shows that captopril tablets of the present invention have a very high stability in moisture-proof "cold blister" packaging.

Example 1

(comparative Example)

(Effect of the ratio of lactose monohydrate and microcrystalline cellulose on the compressibility of the powder blends)

Composition (mg/tablet) (g/5000 tablets)

| | № of experiment | | | | | |
|---|---|---|---|---|---|---|
| | 1/1 | | 1/2 | | 1/3 | |
| | (mg) | (g) | (mg) | (g) | (mg) | (g) |
| Captopril | 25.0 | 125.0 | 25.0 | 125.0 | 25.0 | 125.0 |
| Lactose monohydrate | 85.0 | 425.0 | 70.0 | 350.0 | 60.0 | 300.0 |
| Microcrystalline cellulose | 25.0 | 125.0 | 40.0 | 200.0 | 50.0 | 250.0 |
| Corn starch | 22.0 | 110.0 | 22.0 | 110.0 | 22.0 | 110.0 |
| Stearine | 3.0 | 15.0 | 3.0 | 15.0 | 3.0 | 15.0 |
| Sum of weights | 160.0 | 800.0 | 160.0 | 800.0 | 160.0 | 800.0 |
| Lactose:microcrystalline cellulose ratio | 3.4:1 | | 1.75:1 | | 1.2:1 | |

The components are sieved through a 0.5 mm sieve and thereafter homogenized in a Lödige 5M type high speed mixer under stirring for 5 minutes. The homogenized powder mixture is pressed to tablets on a Fette E XI type eccentric tabletting machine by using flat rimmed tabletting punches under a pressing force of 5 kN, 10 kN and 15 kN, respectively. The breaking strength (Schleuniger 4M measuring apparatus), friability (Roche-type friability measuring device, 100 r.p. 4 minutes) and the disintegration time at 37°C in distilled water (Erweka disintegration measuring apparatus) of the tablets is determined.

The results are summarized in Table 1.

## Table 1

## Results of tests carried out on the tablets

| | № of experiment | | | | | | | | |
| --- | --- | --- | --- | --- | --- | --- | --- | --- | --- |
| | 1/1 | | | 1/2 | | | 1/3 | | |
| Pressing force | 5 | 10 | 15 | 5 | 10 | 15 | 5 | 10 | 15 |
| Breaking strength (average of 10 tablets, N) | 11 | 35 | 48 | 15 | 41 | 51 | 19 | 46 | 59 |
| Friability (average of 10 tablets, %) | 14 | 1.2 | 0.9 | 8.4 | 0.9 | 0.6 | 6.3 | 0.8 | 0.4 |
| Disintegration time (average of 6 tablets, s) | 5 | 15 | 35 | 5 | 20 | 30 | 5 | 20 | 45 |

The above data show that on changing the ratio of lactose and microcrystalline cellulose in favour of microcrystalline cellulose the pressability of the powder mixture improves (the breaking strength values corresponding to identical pressing force increase and the friability decrease) and the disintegration time of the tablets is very good (shorter than 1 minute). If the mixture contains lactose and microcrystalline cellulose in approximately identical amounts (Experiment № 1/3) the friability is below 0.5 % and this is a characteristic feature of tablets having excellent mechanical strength.

In the above Experiment № 1/3 corresponds to the composition of the present invention.

Example 2

(comparative Example)

(Effect of the ratio of lactose monohydrate and microcrystalline cellulose on the moisture-uptake of tablets) Composition (mg/tablet) (g/1000 tablets)

| | № of composition | | | | |
|---|---|---|---|---|---|
| | 2/1 | 2/2 | 2/3 | 2/4 | 2/5 |
| Captopril | 100.0 | 100.0 | 100.0 | 100.0 | 100.0 |
| Lactose monohydrate | 330.0 | 270.0 | 210.0 | 150.0 | 90.0 |
| Microcrystalline cellulose | 60.0 | 120.0 | 180.0 | 240.0 | 300.0 |
| Corn starch | 60.0 | 60.0 | 60.0 | 60.0 | 60.0 |
| Cutina H* | 5.60 | 5.60 | 5.60 | 5.60 | 5.60 |
| Aerosil 200** | 2.80 | 2.80 | 2.80 | 2.80 | 2.80 |
| Magnesium-stearate | 1.60 | 1.60 | 1.60 | 1.60 | 1.60 |
| Sum of weights | 560.0 | 560.0 | 560.0 | 560.0 | 560.0 |

Remarks:

\* = hydrogenated castor oil

\*\* = colloidal silica

The tablets are prepared by using the method and equipment disclosed in Example 1; batch size: 1000 tablets. The tablets are pressed by using tabletting punches and dye having a diameter of 12 mm and under a pressing force of 20 kN.

Moisture-uptake is determined by storing the tablets in an air-conditioner (LABORMIM, LP-23) at 40°C and a relative humidity content of 75 %. (The relative humidity content of 75 % is provided by a saturated solution of ammonium sulfate; the fluctuation of temperature and humidity is controlled by a thermometer-hygrometer - "LOMBIK HÖMÉRÖ és ÜVEGIPARI MÜSZERGYÁRTÓ SZÖVETKEZET" type 06912). During the storing period of two weeks the temperature fluctuated between 39.7°C and 40.4°C, while the humidity varied between 73 % and 88 %.

Moisture-uptake of the tablets is measured after a storing period of 1, 2, 4, 8 and 14 days, respectively. Moisture-uptake is determined on the basis of the weight increase of the tablets (SARTORIUS A 200 S analytic balance) and calculated with the aid of the following equation:

$$\text{Moisture-uptake w/w \%} = \frac{m_2 - m_1}{m_1} \times 100$$

wherein

$m_1$ = weight of the sample before storage
$m_2$ = weight of the sample after storage

Not only the tablets, but also the components used for the preparation thereof were placed in the air conditioner and moisture-uptake of the captopril (active ingredient) and the auxiliary agents was measured at the points of time indicated.

The results are summarized in Tables 2 and 3.

## Table 2

## Moisture-uptake of tablets, w/w %

| № of compo-sition | Lactose: microcrys-talline cellulose ratio | Storing period (days) | | | | | |
|---|---|---|---|---|---|---|---|
| | | 0 | 1 | 2 | 4 | 8 | 14 |
| 2/1 | 5.5:1 | 0.0 | 0.27 | 0.37 | 0.31 | 0.27 | 0.35 |
| 2/2 | 2.25:1 | 0.0 | 0.33 | 0.45 | 0.37 | 0.34 | 0.36 |
| 2/3 | 1.17:1 | 0.0 | 0.43 | 0.56 | 0.48 | 0.45 | 0.43 |
| 2/4 | 0.625:1 | 0.0 | 0.51 | 0.66 | 0.54 | 0.53 | 0.50 |
| 2/5 | 0.3:1 | 0.0 | 0.66 | 0.82 | 0.68 | 0.67 | 0.63 |

## Table 3

## Moisture-uptake of the active ingredient and the auxiliary

## agents (w/w %)

| Component | Storing time (days) | | | | | |
|---|---|---|---|---|---|---|
| | 0 | 1 | 2 | 4 | 8 | 14 |
| Captopril | 0.0 | 0.0 | 0.0 | 0.0 | 0.0 | 0.0 |
| Lactose monohydrate | 0.0 | <0.01 | <0.01 | 0.01 | 0.02 | 0.02 |
| Microcrystalline cellulose | 0.0 | 0.94 | 1.28 | 1.15 | 1.07 | 1.24 |
| Corn starch | 0.0 | 1.44 | 1.82 | 1.58 | 1.49 | 1.58 |
| Cutina H | 0.0 | 0.0 | 0.0 | 0.0 | 0.0 | 0.0 |
| Aerosil 200 | 0.0 | 0.45 | 0.68 | 0.60 | 0.80 | 0.92 |
| Magnesium-stearate | 0.0 | 0.12 | 0.21 | 0.19 | 0.20 | 0.22 |

The above data show that on augmenting the amount of microcrystalline cellulose in the tablets the moisture-uptake of the tablet increases and this is in conformity with the finding that during storage lactose monohydrate does not take up moisture while the moisture-uptake of microcrystalline cellulose is above 1 w/w %. Since the high moisture-uptake of microcrystalline cellulose is known from prior art, the moisture-uptake of the tablets is in conformity with the expected results.

In the above Experiment composition № 2/3 corresponds to the present invention.

Example 3

(comparative Example)

(Effect of the ratio of lactose monohydrate and microcrystalline cellulose on the stability of captopril)

In this experiment the captopril active ingredient content of the tablets is characterized by determining the captopril disulfide content of the tablets before and after storing (the conditions of storing are those disclosed in Example 2). The amount of captopril disulfide in the tablets is determined according to the HPLC methods described in USP 23. To the measurement VARIAN MODEL 5000 Liquid Chromatograph type apparatus, PYE UNICAM LC-3 UV detector and HEWLETT-PACKARD HP 3394A integrator is used. The disulfide content of the tablets is expressed in the % by weight of the nominal captopril content of the tablets. The results are summarized in Table 4.

## Table 4

### Amount of captopril disulfide in the tablets, expressed in % by weight of the nominal captopril content of the tablets (w/w %)

| № of compo-sition | Lactose: microcrys-talline cellulose ratio | Storing time (days) | | | | | |
|---|---|---|---|---|---|---|---|
| | | 0 | 1 | 2 | 4 | 8 | 14 |
| 2/1 | 5.5:1 | 0.28 | 0.46 | 0.73 | 0.95 | 2.13 | 5.15 |
| 2/2 | 2.25:1 | 0.30 | 0.45 | 0.62 | 0.91 | 1.39 | 3.47 |
| 2/3 | 1.17:1 | 0.28 | 0.46 | 0.56 | 0.91 | 1.39 | 2.69 |
| 2/4 | 0.625:1 | 0.34 | 0.56 | 0.78 | 1.05 | 1.55 | 3.25 |
| 2/5 | 0.3:1 | 0.34 | 0.62 | 0.73 | 1.02 | 1.96 | 3.60 |

It appears from the above data that on increasing the microcrystalline cellulose content of the tablets (i.e. decreasing the lactose content) the amount of captopril disulfide formed in the tablets passes a minimum value i.e. decomposition of the composition (disulfide formation) is the lowest at a well-defined ratio of lactose monohydrate and microcrystalline cellulose. This experimental result is surprising because according to the teaching of prior art a greater rate of oxidation of captopril - i.e. the formation of a higher amount of captopril disulfide - could have been expected in a system having higher moisture content, i.e. at a higher microcrystalline cellulose content.

Example 4

(comparative Example)

(Effect of the ratio of lactose monohydrate and microcrystalline cellulose on the stability of captopril)

In order to determine more precisely the optimal ratio, in this series of experiments the stability of the captopril content of the tablets is determined by using lactose/microcrystalline ratios near to those found to be advantageous according to Examples 2 and 3. The composition of the tablets is adjusted to values between the compositions 2/2 and 2/4. The compositions used are disclosed in Table 5. Stability is determined before and after a storing period of 2 weeks and 4 weeks, respectively, by measuring the captopril disulfide content of the tablets according to the HPLC method disclosed in Chapter "Captopril Tablets" of USP 23. To the measurements a VARIAN MODEL 5000 Liquid Chromatograph type apparatus, a PYE UNICAM LC-3 UV detector and a HEWLETT PACKARD HP 3394A integrator is used. The amount of disulfide measured in the tablets is expressed as % weight of the nominal captopril content of the tablets. The results are summarized in Table 5.

## Table 5

### Composition (mg/tablet)    (g/1000 tablets)

| | № of composition | | | | |
|---|---|---|---|---|---|
| | 4/1 | 4/2 | 4/3 | 4/4 | 4/5 |
| Lactose:microcrystalline cellulose ratio | 2:1 | 1.6:1 | 1.25:1 | 1:1 | 0,7:1 |
| Captopril | 100.0 | 100.0 | 100.0 | 100.0 | 100.0 |
| Lactose monohydrate | 260.0 | 234.0 | 217.0 | 195.0 | 160.0 |
| Microcrystalline cellulose | 130.0 | 146.0 | 173.0 | 195.0 | 230.0 |
| Corn starch | 60.0 | 60.0 | 60.0 | 60.0 | 60.0 |
| Cutina H* | 5.60 | 5.60 | 5.60 | 5.60 | 5.60 |
| Aerosil 200** | 2.80 | 2.80 | 2.80 | 2.80 | 2.80 |
| Magnesium-stearate | 1.60 | 1.60 | 1.60 | 1.60 | 1.60 |
| Average weight | 560.0 | 560.0 | 560.0 | 560.0 | 560.0 |

Remarks:

   * = hydrogenated castor oil

   ** = colloidal silica

The tablets are prepared by using the method and apparatus described in Example 1; batch size 1000 tablets. On pressing the tablets with tabletting punches and dye having a diameter of 12 mm and a pressing force of 12 kN is used.

Moisture uptake is carried out by storing the tablets in an air conditioner (LABORMIM, LP-23) at 40°C and a relative humidity of 75 %. (The relative humidity content of 75 % is provided by a saturated solution of ammonium sulfate: the fluctuation of temperature and humidity is controlled by a thermometer-hygrometer - "LOMBIK HÖMÉRÖ és ÜVEGIPARI MÜSZERGYÁRTÓ SZÖVETKEZET" type 06912). During the storing period of two weeks the temperature fluctuated between 39.6°C and 40.5°C, while the humidity varied between 72 % and 78 %.

Moisture-uptake is determined after a storing period of 4 weeks. Moisture-uptake is determined on the basis of the weight increase of the tablets (SARTORIUS A 200 S analytical balance) and calculated with the aid of the following equation:

$$\text{Moisture-uptake w/w \%} = \frac{m_2 - m_1}{m_1} \times 100$$

wherein

$m_1$ = weight of the sample before storage
$m_2$ = weight of the sample after storage

The results are summarized in Table 6.

## Table 6

| | №. of composition | | | | |
|---|---|---|---|---|---|
| | 4/1 | 4/2 | 4/3 | 4/4 | 4/5 |
| Ratio of lactose and microcrystalline cellulose | 2.0:1 | 1.6:1 | 1.25:1 | 1.0:1 | 0.7:1 |
| Storing for 2 weeks captopril disulfide (%) | 2.5 | 2.4 | 2.2 | 1.7 | 2.1 |
| Storing for 4 weeks captopril disulfide (%) | 5.3 | 5.3 | 4.4 | 4.1 | 4.9 |
| Moisture uptake (%) | 0.4 | 0.5 | 0.6 | 0.6 | 0.9 |

The results of this experiment fully confirm the data obtained according to Example 3. Thus on increasing the microcrystalline cellulose content of the tablets (i.e. decreasing the lactose content) the amount of captopril disulfide formed in the tablets on storing reaches a minimum value and at a specified lactose monohydrate : microcrystalline cellulose ratio the decomposition of the tablet (disulfide formation) shows a minimum. The results of these two series of experiments are surprising and unforeseen because on the basis of prior art one could expect an oxidation of higher extent of captopril (i.e. the formation of a larger amount of captopril disulfide) in a system of higher moisture content. One could expect that the higher the microcrystalline cellulose content, the larger degree of captopril decomposition would take place and the higher amount of captopril disulfide would be formed. However if the ratio of lactose and microcrystalline cellulose is between 1.5:1 and 1:1 a surprising surplus effect takes place.

### Example 5

Tablets having an active ingredient content of 12.5 mg Batch size: 2 million tablets (140 kg)

Substances used:

| Component | Amount, kg |
|---|---|
| Captopril (particle size: 90 % between 3.5 and 46.9 µm) | 25.00 |
| Lactose monohydrate | 52.50 |
| Microcrystalline cellulose | 45.00 |
| Corn starch | 15.00 |

(continued)

| Component | Amount, kg |
|---|---|
| Hydrogenated castor oil | 1.40 |
| Colloidal silica | 0.70 |
| Magnesium stearate | 0.40 |

The above substances - with the exception of magnesium stearate - are stirred in a 450 l mixer for 20 minutes, whereupon the magnesium stearate is added and stirring is continued for further 2 minutes. Tabletting is carried out with a Fette Perfecta 2000 type tabletting machine (43 pressing sites) by using flat rimmed split punches at 40 r.p.m. The breaking strength of the tablets is 35-45 N, the disintegration time amounts to 2-3 minutes, the active ingredient is dissolved within 5 minutes at a rate of 100 % and the relative standard deviation (RSD) of the individual active ingredient content is 3.7 %. On storing the tablets for 3 months at 40°C in a glass container closed with a polyethylene cap the amount of captopril disulfide formed is 1.6 %, while the breaking strength, disintegration time and the velocity of active ingredient dissolution remained substantially unchanged. On the basis of the above data the captopril containing tablets prepared according to this Example fully meet the requirements of the Pharmacopoeia (USP 23, Captopril Tablets).

In the tablets the ratio of lactose and microcrystalline cellulose amounts to 1.16:1.

Example 6

Tablets having an active ingredient content of 25 mg Batch size: 100,000 tablets (14 kg)

Substances used:

| Component | Amount, kg |
|---|---|
| Captopril (particle size: 90 % between 5.5 and 69.6 $\mu$m) | 2.50 |
| Lactose monohydrate | 4.90 |
| Microcrystalline cellulose | 4.90 |
| Corn starch | 1.50 |
| Hydrogenated castor oil | 0.10 |
| Colloidal silica | 0.06 |
| Magnesium stearate | 0.04 |

The above substances - with the exception of magnesium stearate - are stirred in a 50 l mixer for 20 minutes, whereupon the magnesium stearate is added and stirring is continued. Tabletting is carried out on a Manesty B3B type tabletting machine having 16 pressing sites, by using flat rimmed twice split press punches at 30 r.p.m. The breaking strength of the tablets amounts to 40-55 N, the disintegration time amounts to 2-3 minutes, the dissolution of the active ingredient is 100 % within 10 minutes. The relative standard deviation (RSD) of the individual tablets is 1.9 %. On storing the tablets in a glass container closed with a polyethylene cap at 40°C for 3 months the amount of the captopril disulfide is 1.5 % while the breaking strength, disintegration time of the tablets and the dissolution speed of the active ingredient remain substantially unchanged. On the basis of the above data the captopril containing tablets prepared according to this Example fully meet the requirements of the Pharmacopoeia (USP 23, Captopril Tablets).

Example 7

Tablets having an active ingredient content of 50 mg

Batch size: 50,000 (14 kg)

Substances used:

| Component | Amount, kg |
|---|---|
| Captopril (particle size: 90 % between 2.3 and 36.5 µm) | 2.50 |
| Lactose monohydrate | 5.85 |
| Microcrystalline cellulose | 3.90 |
| Corn starch | 1.50 |
| Hydrogenated castor oil | 0.14 |
| Colloidal silica | 0.07 |
| Magnesium stearate | 0.04 |

The above components - with the exception of magnesium stearate - are stirred in a 50 l mixer for 20 minutes, whereupon the magnesium stearate is added and stirring is continued for a further 2 minutes. Tabletting is performed on a Manesty BB3B type tabletting machine having 27 press sites, by using flat rimmed twice split press punches, at 30 r.p.m. The breaking strength of the tablets amounts to 45-65 N, the disintegration time is 2-3 minutes, the dissolution of the active ingredient within 10 minutes is 100 % and the relative standard deviation of the individual active ingredient content of the tablets (RSD) amounts to 3.7 %. On storing the tablets in a glass container closed with a polyethylene cap at 40°C for 3 months the amount of the captopril disulfide formed in the tablets is 1.7 %, while the breaking strength and disintegration time of the tablets and the speed of dissolution of the active ingredient remain substantially unchanged. On the basis of the above data the captopril containing tablets prepared according to this Example fully meet the requirements of the Pharmacopoeia (USP 23, Captopril Tablets).
In the tablets the ratio of the lactose and microcrystalline cellulose is 1.5:1.

Example 8

Tablets having an active ingredient content of 100 mg Batch size 500,000 tablets (140 kg)

Substances used:

| Component | Amount, kg |
|---|---|
| Captopril (particle size: 90 % between 4.3 and 65.3µm) | 25.00 |
| Lactose monohydrate | 52.50 |
| Microcrystalline cellulose | 45.00 |
| Corn starch | 15.00 |
| Hydrogenated castor oil | 1.40 |
| Colloidal silica | 0.70 |
| Magnesium stearate | 0.40 |

The above substances - with the exception of magnesium stearate - are stirred in a 450 l mixer for 20 minutes whereupon the magnesium stearate is added and stirring is continued for further 2 minutes. Tabletting is carried out on a Manesty BB3B type tabletting machine having 35 pressing sites, by using flat rimmed split punches having a diameter of 12 mm at 30 r.p.m. The breaking strength of the tablets is 70-90 N, the disintegration time is 1.5-3 minutes, the dissolution of the active ingredient within 10 minutes is above 90 % and the relative standard deviation of the individual active ingredient content of the tablets (RSD) is 3.7 %. On storing the tablets in a glass container closed with a polyethylene cap at a temperature of 40°C for 3 months the amount of captopril disulfide formed is 1.7 % while the breaking strength and dissolution time of the tablets and the dissolution speed of the active ingredient remains substantially unchanged. On the basis of the above data the captopril containing tablets prepared according to this Example fully meet the requirements of the Pharmacopoeia (USP 23, Captopril Tablets).

In the tablets the ratio of lactose and microcrystalline cellulose is 1.16:1.

Example 9

Determination of the standard deviation of the active ingredient content of tablets according to the present invention on halving and dividing the tablets into quarters.

The test is carried out by using cross-split tablets having an active ingredient content of 25 mg. In order to administer a dose of 12.5 mg and 6.25 mg, respectively, the patients have to cut the tablets into two or four parts, respectively, during the treatment.

In the course of the test the standard deviation of the active ingredient content of the whole tablets is determined by measuring the individual active ingredient content of 10 tablets each and the relative standard deviation of the active ingredient content (RSD) is calculated. Thereafter 10 tablets from both compositions each are broken in two parts by hand and the relative standard deviation of the weight and active ingredient content of the halved tablets is determined by measuring alternately the weight and captopril content of the half-tablets remaining in the right and left hand, respectively. The remaining half tablets are then broken by hand in two parts and the relative standard deviation of the tablets broken into four parts is determined by measuring alternately the weight and captopril content of the quarter tablets remaining in the right and left hand, respectively.

The captopril content of the whole, halved and quarter tablets is determined according to the Chapter "Captopril Tablets" of USP 23 by measuring the individual active ingredient content of the captopril tablets by HPLC.

The results are summarized in Table 7.

Table 7

| Tested tablet | Weight RSD % | Active ingredient content RSD % |
|---|---|---|
| Whole | 2.5 | 2.0 |
| Half | 8.4 | 8.2 |
| Quarter | 13.4 | 12.9 |

The above data show that on halving and breaking the tablets in four parts, respectively, the standard deviation of the weight and active ingredient content of the half and quarter tablet parts is lower than the 15 % limit allowed for the standard deviation of the active ingredient content. Accordingly the tablets of the present invention can be preferably used in therapeutical practice if tablets are to be broken to two or four parts in order to adjust the optimal dose of treatment.

Example 10

(comparative Example)

In comparative Example 10 tablets according to the present invention (Example 2, № 2/3) are compared with those having a lactose/microcrystalline cellulose ratio outside the scope of the invention; otherwise the active ingredient content (12.5 mg) and the other components of the tablets are identical. The tablets are packed into blister (bed formed from a PVC/PVDC plastic foil and closed with an aluminium foil). The samples are subjected to loaded stability testing and the disulfide content is measured. The tablets packed into blister are stored at 40°C and a relative humidity content of 75 % for 3 months. The captopril disulfide content of the tablets is determined before and after storing. The results are summarized in Table 8.

Table 8

| Sample | Captopril disulfide content | |
|---|---|---|
| | before storing | after storing |
| Present invention, Example 2, № 2/3 | 0.31 % | 3.32 % |
| Reference sample | 0.30 % | 5.04 % |

Example 11

Testing of the stability of the tablets of the present invention (active ingredient content 12.5 mg) packed in moisture proof "cold blister".

The tablets of the present invention (Example 2, № 2/3: active ingredient content 12.5 mg) are filled into beds formed from aluminium foil combined with plastic (Al/PE/PVC) by using a deep-drawing packaging machine. The beds are closed by welding with an aluminium foil.
The tablets are stored at 40°C (± 2°C) and a relative humidity content of 75 % for 3 months whereupon the captopril disulfide content of the tablets is determined by HPLC method in accordance with USP 23.
The results obtained are summarized in Table 9.

Table 9

| Sample | Captopril disulfide content | |
|---|---|---|
| | before storing | after storing |
| Example 2, № 2/3 | 0.19 % | 0.62 % |

It can be seen that the captopril disulfide content of the tablets increases only to small extent, well below the allowed limit, even if stored under agressive conditions.

**Claims**

1. Captopril tablets comprising a filler and binding agent, disintegrating agent, a lubricant and a glidant agent **wherein** the filler and binding agent is a 1.5:1 - 1:1 weight ratio mixture of lactose and microcrystalline cellulose and the total amount of said mixture is 60-80 % by weight of the total weight of the tablet.

2. Tablets according to Claim 1 **wherein** the total amount of the mixture of lactose and microcrystalline cellulose is 67-75 % by weight of the total weight of the tablets.

3. Tablets according to Claim 2 **wherein** the total amount of the mixture of lactose and microcrystalline cellulose is 69-70 % by weight of the total weight of the tablets.

4. Tablets according to any of Claims 1-3 **wherein** the disintegrating agent is corn starch, carboxymethyl starch, partially hydrolized starch, carboxymethyl cellulose or cross-linked polyvinyl pyrrolidone, the amount thereof being 1-20 % by weight of the total weight of the tablets.

5. Tablets according to any of Claims 1-3 **wherein** the lubricant is stearine, magnesium stearate, calcium stearate, sodium stearyl fumarate or hydrogenated castor oil, in an amount of 0.2-3 % by weight of the total weight of the tablets.

6. Tablets according to any of Claims 1-3 **wherein** the glidant agent is colloidal silica in an amount of 0.2-1 % by weight of the total weight of the tablets.

7. Tablets according to any of Claims 1-3 **wherein** the lactose is lactose monohydrate having an average particle size of 50-200 μm.

8. Tablets according to Claim 7 **wherein** the lactose is lactose monohydrate having an average particle size of 80-160 μm.

9. Tablets according to any of Claims 1-3 **wherein** 90 % of the microcrystalline cellulose has an average particle size of 20-150 μm.

10. Tablets according to Claim 9 **wherein** 90 % of the microcrystalline cellulose has an average particle size of 50-100 μm.

**11.** Process for the preparation of captopril tablets containing a filler and binding agent, a disintegrating agent, a lubricant and a glidant agent **which comprises** using as filler and binding agent a 1.5:1 - 1:1 mixture of lactose and microcrystalline cellulose, the amount of said mixture being 60-80% by weight of the total weight of the tablets.

**12.** Process according to Claim 11 **which comprises** using lactose, microcrystalline cellulose, disintegrating agents, lubricant and/or glidant agents defined in any of Claims 2-10.

**Patentansprüche**

**1.** Captopril-Tabletten, umfassend ein(en) Füllstoff und Bindemittel, den Zerfall förderndes Mittel, ein Lubrikans und ein Gleitmittel, wobei der Füllstoff und das Bindemittel eine Mischung im Gewichtsverhältnis von 1,5:1 - 1:1 aus Lactose und mikrokristalliner Cellulose ist und die Gesamtmenge der Mischung 60 - 80 Gew.-% des Gesamtgewichtes der Tablette beträgt.

**2.** Tabletten gemäß Anspruch 1, wobei die Gesamtmenge der Mischung aus Lactose und mikrokristalliner Cellulose 67 - 75 Gew.-% des Gesamtgewichtes der Tabletten beträgt.

**3.** Tabletten gemäß Anspruch 2, wobei die Gesamtmenge der Mischung aus Lactose und mikrokristalliner Cellulose 69 - 70 Gew.-% des Gesamtgewichtes der Tabletten beträgt.

**4.** Tabletten gemäß mindestens einem der Ansprüche 1 - 3, wobei das den Zerfall fördernde Mittel Maisstärke, Carboxymethylstärke, partiell hydrolisierte Stärke, Carboxymethylcellulose oder vernetztes Polyvinylpyrrolidon ist, wobei die Menge davon bei 1 - 20 Gew.-% des Gesamtgewichtes der Tabletten liegt.

**5.** Tabletten gemäß mindestens einem der Ansprüche 1 - 3, wobei das Lubrikans Stearin, Magnesiumstearat, Calciumstearat, Natriumstearylfumarat oder hydriertes Castoröl in einer Menge von 0,2 - 3 Gew.-% des Gesamtgewichtes der Tabletten ist.

**6.** Tabletten gemäß mindestens einem der Ansprüche 1 - 3, wobei das Gleitmittel kolloidales Silika in einer Menge von 0,2 - 1 Gew.-% des Gesamtgewichtes der Tabletten ist.

**7.** Tabletten gemäß mindestens einem der Ansprüche 1 - 3, wobei die Lactose Lactosemonohydrat mit einer mittleren Teilchengröße von 50 - 200 µm ist.

**8.** Tabletten gemäß mindestens Anspruch 7, wobei die Lactose Lactosemonohydrat mit einer mittleren Teilchengröße von 80 - 160 µm ist.

**9.** Tabletten gemäß mindestens einem der Ansprüche 1 - 3, wobei 90 % der mikrokristallinen Cellulose eine mittlere Teilchengröße von 20 - 150 µm aufweist.

**10.** Tabletten gemäß Anspruch 9, wobei 90 % der mikrokristallinen Cellulose eine mittlere Teilchengröße von 50 - 100 µm aufweist.

**11.** Verfahren zur Herstellung von Captopril-Tabletten, enthaltend ein(en) Füllstoff und Bindemittel, ein den Zerfall förderndes Mittel, ein Lubrikans und ein Gleitmittel, welches das Verwenden einer 1,5:1 - 1:1-Mischung aus Lactose und mikrokristalliner Cellulose als Füllstoff und Bindemittel umfasst, wobei die Menge der Mischung 60 - 80 Gew.-% des Gesamtgewichtes der Tabletten beträgt.

**12.** Verfahren gemäß Anspruch 11, welches die Verwendung von Lactose, mikrokristalliner Cellulose, von den Zerfall fördernden Mitteln, Lubrikans und/oder Gleitmitteln, welche in mindestens einem der Ansprüche 2 - 10 definiert sind, umfasst.

**Revendications**

**1.** Comprimés de captopril comprenant une charge et agent liant, un agent de délitescence, un lubrifiant et un agent lissant, dans lesquels la charge et agent liant sont un mélange dans un rapport pondéral compris entre 1,5/1 et 1/1 de lactose et de cellulose microcristalline, et dans lesquels la quantité totale dudit mélange constitue 60 à 80 % en poids, du poids total du comprimé.

2. Comprimés selon la revendication 1, dans lesquels la quantité totale de mélange lactose/cellulose microcristalline constitue 67 à 75 % en poids, du poids total des comprimés.

3. Comprimés selon la revendication 2, dans lesquels la quantité totale de mélange lactose/cellulose microcristalline constitue 69 à 70 % en poids, du poids total des comprimés.

4. Comprimés selon l'une quelconque des revendications 1 à 3, dans lesquels l'agent de délitescence est l'amidon de maïs, le carboxyméthylamidon, l'amidon partiellement hydrolysé, la carboxyméthylcellulose ou la polyvinylpyrrolidone réticulée, et dont la quantité constitue 1 à 20 % en poids, du poids total des comprimés.

5. Comprimés selon l'une quelconque des revendications 1 à 3, dans lesquels le lubrifiant est la stéarine, la stéarate de magnésium, le stéarate de calcium, le fumarate de stéaryle et de sodium ou l'huile de ricin hydrogénée, présente en quantité constituant 0,2 à 3 % en poids, du poids total des comprimés.

6. Comprimés selon l'une quelconque des revendications 1 à 3, dans lesquels l'agent lissant est une silice colloïdale présente en quantité constituant 0,2 à 1 % en poids, du poids total des comprimés.

7. Comprimés selon l'une quelconque des revendications 1 à 3, dans lesquels le lactose est du lactose monohydraté ayant une taille moyenne de particules de 50 à 200 $\mu$m.

8. Comprimés selon la revendication 7, dans lesquels le lactose est du lactose monohydraté ayant une taille moyenne de particules de 80 à 160 $\mu$m.

9. Comprimés selon l'une quelconque des revendications 1 à 3, dans lesquels 90 % de la cellulose microcristalline ont une taille moyenne de particules de 20 à 150 $\mu$m.

10. Comprimés selon la revendication 9, dans lesquels 90 % de la cellulose microcristalline ont une taille moyenne de particules de 50 à 100 $\mu$m.

11. Procédé pour la préparation de comprimés de captopril contenant une charge et agent liant, un agent de délitescence, un lubrifiant et un agent lissant, qui comprend l'utilisation d'une charge et agent liant sous la forme d'un mélange dans un rapport pondéral compris entre 1,5/1 et 1/1 de lactose et de cellulose microcristalline, la quantité dudit mélange constituant 60 à 80 % en poids, du poids total des comprimés.

12. Procédé selon la revendication 11, qui comprend l'utilisation de lactose, de cellulose microcristalline, d'agents de délitescence, de lubrifiant et/ou d'agents lissants selon l'une quelconque des revendications 2 à 10.